# EUROPEAN PATENT SPECIFICATION

(11) **EP 1 835 888 B1**
(45) Date of publication and mention of the grant of the patent: **12.07.2017**
(21) Application number: 06702900.9
(22) Date of filing: 12.01.2006
(51) Int. Cl.: A61K 9/19, A61K 47/28, A61K 47/36, A61K 31/337

(54) **CHOLANIC ACID-CHITOSAN COMPLEX FORMING SELF-AGGREGATES AND PREPARATION METHOD THEREOF**
SELBSTAGGREGATE BILDENDER CHOLANSÄURE-CHITOSAN-KOMPLEX UND HERSTELLUNGSVERFAHREN
COMPLEXE ACIDE CHOLANIQUE-CHITOSANE FORMANT AUTOMATIQUEMENT DES AGREGATS ET SON PROCEDE DE PREPARATION

(30) Priority: 14.01.2005 KR 20050003856
(43) Date of publication of application: 26.09.2007
(73) Proprietor: Korea Institute of Science and Technology, Seoul 136-792 (KR)
(72) Inventor: KWON, Ick Chan, Seoul 139-220 (KR); JEONG, Seo Young, Goyang-si, Gyunggi-do, 410-837 (KR); KIM, In-san, Daegu 706-140 (KR); CHUNG, Hesson, Gangseo-gu 157-886 (KR); SEO, Sang Bong Jakwang Co., Ltd., Ansung-si 456-380 (KR); PARK, Jae Hyung, Dongan-gu Anyang-si, Gyunggi-do 431-060 (KR); KWON, Seunglee, Ulsan 680-837 (KR); KIM, Kwangmyung, Seoul 152-092 (KR); KIM, Jong-ho, Seoul 122-932 (KR)
(74) Representative: advotec.
(86) International application number: PCT/KR2006/000130
(87) International publication number: WO 2006/075881

(56) References cited:
- KR-A- 2005 008 323
- US-A1- 2003 235 916
- PARK J H ET AL: "Self-assembled nanoparticles based on glycol chitosan bearing 5beta-cholanic acid for RGD peptide delivery", JOURNAL OF CONTROLLED RELEASE, ELSEVIER, AMSTERDAM, NL, vol. 95, no. 3, 24 March 2004 (2004-03-24) , pages 579-588, XP004496396, ISSN: 0168-3659, DOI: DOI:10.1016/J.JCONREL.2003.12.020
- LEE K Y ET AL: "Self-aggregates of deoxycholic acid-modified chitosan as a novel carrier of adriamycin", COLLOID POLYM SCI, vol. 278, 11 July 2000 (2000-07-11), pages 1216-1219, XP002622441,
- KWON S ET AL: "Physicochemical characteristics of self-assembled nanoparticles based on glycol chitosan bearing 5beta-cholanic acid", LANGMUIR, vol. 19, 28 October 2003 (2003-10-28), pages 10188-10193, XP002622442,
- ZHU AND NICHIFOR: "Polymeric materials containing bile acids", ACC. CHEM. RES., vol. 35, 6 December 2002 (2002-12-06), pages 539-546, XP002622443,
- SON ET AL.: 'Preparation of a hydrophobized chitosan oligosaccharide for application as an efficient gene carrier' MACROMOLECULAR RESEARCH vol. 12, no. 6, 2004, pages 573 - 580, XP008120066
- THONGNGAM ET AL.: 'Influence of pH, ionic strength, and temperature on self-association and interaction of sodium dodecyl sulfate in the absence and presence of chitosan' LANGMUIR vol. 21, no. 1, 04 January 2005, pages 79 - 86, XP008119937

## Description

### Technical Field

The present invention relates to a pharmaceutical formulation of cholanic acid-chitosan complex incorporated with paclitaxel and preparation method thereof. The cholanic acid-chitosan complex composing of hydrophobic cholanic acid and hydrophilic chitosan forms self-aggregates in an aquatic system.

### Background Art

Anticancer chemotherapy began to develop when methotrexate was found to completely cure choriocarcinoma. About fifty anticancer drugs are currently available and exhibit good therapeutic efficacy when administered to treat choriocarcinoma, leukemia, Wilms tumors, Ewing's sarcoma, rhabdomyosarcoma, retinoblastoma, lymphoma, mycosis fungoides, and testicular cancer.

With the recent advance in knowledge on carcinogenesis and characteristics of tumor cells, many studies have concentrated on the development of new anticancer drugs. Anticancer drugs mostly display anticancer effects by inhibiting the synthesis of nucleic acids, which are the basic building blocks of cellular genetic material, or directly binding to nucleic acids, impeding the function of nucleic acids. However, since these anticancer drugs damage normal cells as well as tumor cells, in particular, actively dividing tissue cells, they have side effects including impaired bone marrow function, damage to gastrointestinal tract mucosa, and hair loss.

Chemotherapy for cancer has very limited applicability due to the side effects of anticancer drugs. As described above, since side effects occur due to the toxicity of anticancer drugs affecting normal cells in addition to tumor cells, various studies have been conducted in order to reduce such side effects. Representative methods for reducing such side effects involve using an anticancer drug in a form linked with a polymer and employing micelles or microspheres as carriers for anticancer drugs.

The first method involves linking an anticancer drug with a polymer to provide an anticancer drug-polymer complex. Anticancer drugs have side effects since they have insufficient selectivity for tumor tissue, and thus injure not only tumor cells but also normal cells. Thus, many studies have been focused on the development of methods to effectively deliver anticancer drugs only to the tumor tissue in order to reduce such side effects of anticancer drugs. A representative method employs a polymeric carrier. This method has the following advantages: the *in vivo* distribution of anticancer drug-polymer complexes varies depending on the nature of the polymeric carrier; the anticancer drug has an extended serum half-life; and the release of the anticancer drug can be controlled by regulating the nature of chemical binding between the anticancer drug and the carrier.

The second method employs micelles or microspheres as a carrier for an anticancer drug. With this method, the side effects of anticancer therapy can be minimized by encapsulation an anticancer drug in micelles or microspheres, thus enabling the sustained release of the drug. This method is used for suppressing side effects, which occur when a large amount of an anticancer drug is released and acts within a short period of time, by sustaining the release of the anticancer drug from micelles or microspheres for longer periods of time [Pharm. Res., 1983, 15, 1844].

Recently, a large variety of polymers has been designed and used as polymeric carriers for anticancer drugs, and several organic reactions are used for introducing anticancer drugs into polymeric carriers. In this context, a large number of polymers have been studied, and examples of such polymers include poly(N-2- (hydroxypropyl)methacrylamide), poly(divinyl ether-co-maleic anhydride), poly(styrene-co-maleic anhydride), dextran, poly(ethylene glycol), poly(L-glutamic acid), poly(aspartic acid), and poly(L-lysine).

The anticancer drug-polymer complex-based approach can selectively treat tumor cells using the pathophysiological properties of the tumor tissue that differ from those of normal tissue. Typically, compared to the normal tissue, a larger number of blood vessels forms in the tumor tissue, which requires a greater supply of nutrients. Such tumor vessels are dilated and leaky. The leakiness of tumor vasculature permits macromolecules to extravasate into the tumor tissue in preference to other tissues or organs. Together with the increased vascular permeability, poor lymphatic drainage relative to the normal tissue enables macromolecules to be trapped and accumulate in the tumor tissue. This phenomenon is known as enhanced permeability and retention (EPR) effect [Adv. Drug Deliv. Rev., 2000, 65, 271] . As an attempt using such anticancer drug-polymer complexes, the N-(hydroxypropyl)methacrylamide (HPMA)-anticancer drug complex is currently in Phase II clinical trials [U.S. Pat. No. 5,037,883 (1991)].

In addition, Korean Pat. Registration No. 507968, titled "Anticancer Drug-Chitosan Complex Forming Self- Aggregates and Preparation Method Thereof", describes a technique for forming a complex comprising a hydrophobic anticancer drug and hydrophilic chitosan. In this patent, however, available anticancer drugs are limited specifically to those capable of reacting with amine groups, and the reaction of an anticancer drug with an amine group has the potential to decrease the activity of the anticancer drug.

Chitin, which is a precursor of chitosan, is a major component of the exoskeleton of crustacean, insects and other invertebrates, the cell walls of fungi, and other tissues, and is a linear polymer of N-acetyl-D-glucosamine repeating units, which are joined together by (1->4)-(β-glycosidic linkages. Chitosan is a basic polysaccharide, which is obtained by N-deacetylation, which takes place when chitin is treated with concentrated alkali. In recent years, chitosan has been known to have good properties including bioadhesion, biocompatibility, biodegradability, and formulability, compared to other synthetic polymers.

In this regard, with the aim of overcoming the side effects of conventional carriers for anticancer drugs, the present inventors introduced cholanic acid into a hydrophilic polymer to form self-aggregates, and physically incorporating a hydrophobic anticancer drug into the selfaggregates to provide a cholanic acid-chitosan complex having several advantages of micelles. The present inventors found that the complex traps high concentrations of the hydrophobic anticancer drug, and that along with the improved drug loading capacity, the control of the release of the anticancer drug through the regulation of chemical bonding properties between cholanic acid and chitosan imparts high selectivity for the tumor tissue to the drug-loaded complex.
Modified glycol chitosan self-aggregates loaded with RGD-bearing peptides antagonists of αᵥβ₃ integrin to inhibit the tumor growth by promoting apoptosis of the angiogenetic vascular cells are known (Park J. H. et al: "Self-assembled nanoparticles based on glycol chitosan bearing 5beta-cholanic acid for RGD peptide delivery", JOURNAL OF CONTROLLED RELEASE, ELSEVIER, AMSTERDAM, NL, vol. 95, no. 3,, pages 579-588, ISSN: 0168-3659, DOI: DOI:10.1016/J.JCONREL 2003.12.020, 24 March 2004).

Further, self-aggregates of deoxycholic acid-modified chitosan as carrier of adriamycin are also known from the state of the art Lee K.Y. et al: "Self-aggregates of deoxycholic acid-modified chitosan as a novel carrier of adriamycin", COLLOID POLYM SCI, vol. 278, pages 1216-1219, 11 July 2000).
The modification of glycolchitosan with 5β-cholanic acid is also known from the art Kwon S. et al: "Physicochemical characteristics of self-assembled nanoparticles based on glycol chitosan bearing 5beta-cholanic acid", LANGMUIR, vol. 19, pages 10188-10193, 28 October 2003).

### Disclosure Technical Problem

It is an object of the present invention to provide a pharmaceutical formulation wherein the hydrophobic anticancer drug paclitaxel is incorporated into a cholanic acid-chitosan complex, wherein the cholanic acid- chitosan complex forms self-aggregates, wherein the pharmaceutical formulation shows an increated anticancer effect, an enhanced release control of the anticancer drug paclitaxel as well as less toxicity.

It is another object of the present invention to provide a method of preparing the pharmaceutical formulation characterized in that the hydrophobic anticancer drug paclitaxel is incorporated into the cholanic acid-chitosan complex.

### Technical Solution

In order to accomplish the above objects, the present invention provides a 5-β-cholanic acid-glycol chitosan
complex forming self-aggregates, which allows for the sustained release of paclitaxel for long periods of time with enhanced selectivity for the tumor tissue, and has greatly improved drug loading capacity, thereby having potential for use in anticancer chemotherapy, and a method of preparing the complex. The cholanic acid-chitosan complex forming self-aggregates that further include paxlitaxel.

### Advantageous Effects

The pharmaceutical formulation characterized in that paclitaxel is incorporated into the cholanic acid-chitosan complex, wherein the cholanic acid- chitosan complex of the present invention forms selfaggregates, prolongs the drug release period, enhances the selectivity of the complex for the tumor tissue, and greatly increases drug loading content when a drug is incorporated into the self-aggregates, compared to chemical bonding which entails limits on drug incorporation.Thus, the pharmaceutical formulation of the present invention is useful for anticancer chemotherapy.

### Description of Drawings

FIG. 1 is a graph showing the particle size and distribution of a 5-β-cholanic acid-chitosan complex according to an embodiment of the present invention in an aqueous solution, wherein the particle size and distribution was measured using light scattering;
FIG. 2 is a transmission electron microscope (TEM) image of a 5-β-cholanic acid-chitosan complex according to an embodiment of the present invention;
FIG. 3 is a graph showing the cumulative release of an anticancer drug, paclitaxel, which was incorporated into a 5-β-cholanic acid-chitosan complex according to an embodiment of the present invention;
FIG. 4 is a graph showing the changes in tumor volume of mice to which a 5-β-cholanic acid-chitosan complex containing paclitaxel as an anticancer drug according to an embodiment of the present invention was administered; and
FIG. 5 is a graph showing the changes in body weight of mice to which a 5-β-cholanic acid-chitosan complex containing paclitaxel as an anticancer drug according to an embodiment of the present invention was administered.

### Best Mode

Hereinafter, the present invention will be described in detail.

The present invention provides a pharmaceutical formulation characterized in that paclitaxel is incorporated into the cholanic acid-chitosan complex, wherein the cholanic acid-chitosan complex forms self-aggregates. The anticancer drug carriers for use herein include glycol chitosan preferably having a molecular weight ranging from 10³ to 10^{s} Da. Glycol chitosan having enhanced water solubility due to glycol groups introduced thereinto is used.

5-β-cholanic acid represented by Formula 1, below is used in the preparation of the cholanic acid-chitosan complex of the present invention.

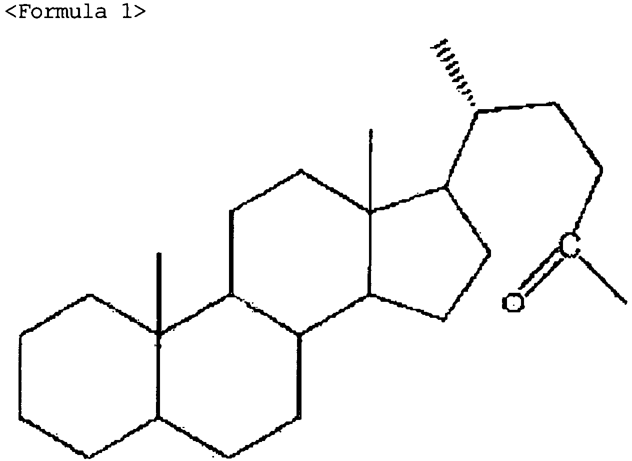

The cholanic acid-chitosan complex of the present invention is able to form micelle-like spherical self-aggregates in an aquatic environment due to its amphiphilic property of having the hydrophobic group of cholanic acid and the hydrophilic group of chitosan.

The size of the cholanic acid-chitosan complex of the present invention preferably ranges from 1 nm to 2,000 nm, and more preferably 10 nm to 800 nm. The size of the cholanic acid-chitosan complex is determined depending on the content of cholanic acid, and the cholanic acid may be contained in an amount from 1 to 70 parts by weight. The cholanic acid-chitosan complex of the present invention further includes an anticancer drug, namely paclitaxel.

Since the inside of the cholanic acid-chitosan complex consists of hydrophobic cholanic acid, and hydrophilic portions of cholanic acid bind to chitosan, the inside of the cholanic acid-chitosan complex of the present invention is highly hydrophobic. Due to this structural property, the cholanic acid-chitosan complex facilitates incorporation of a hydrophobic anticancer drug thereinto. Thus, it is possible to physically incorporate an anticancer drug within the cholanic acid-chitosan complex. Since the incorporated anticancer drug paclitaxel has hydrophobicity, which is a property similar to that of the internal constituent of the cholanic acid-chitosan complex, the cholanic acid-chitosan complex may greatly increase drug loading content, compared to chemical bonding, which imposes limits on drug incorporation, and this enhanced drug loading capacity is much better than that of other carriers.

The size of the cholanic acid-chitosan complex having a trapped anticancer drug according to the present invention ranges preferably from 1 nm to 2,000 nm, and more preferably from 10 nm to 800 nm.

Typically, self-aggregates are spherical aggregates that are formed when amphiphatic molecules having both hydrophilic, and hydrophobic groups spontaneously assemble in an aqueous solution. In such self-assemblies, hydrophilic groups get together on the outer surface, and hydrophobic groups in the inner core [Adv. Drug Deliv. Rev., 1996, 21, 107]. Thus, amphiphilics have been widely used as a system for effectively delivering various anticancer drugs having a hydrophobic character. A drug delivery system using amphiphilic polymers forming such self-aggregates provides sufficient selectivity for target cells and therefore greatly decreased toxicity to normal cells, and enables the sustained release of drugs for long periods of time. Thus, this technique provides a potentially effective new approach in the treatment of serious diseases, such as cancer.

Thus, the pharmaceutical formulation characterized in that the hydrophobic anticancer drug paclitaxel is incorporated into the cholanic acid-chitosan complex of the present invention has a higher selectivity for the tumor tissue due to the enhanced permeability and retention (EPR) effect than general low molecular weight anticancer drugs, and thus accumulates in the tumor tissue in higher amounts. Due to this property, the pharmaceutical formulation of the present invention exhibits effective anticancer activity. Also, since the cholanic acid-chitosan complex spontaneously forms micelle-like spherical self-aggregates in an aqueous solution due to its dual hydrophobic/hydrophilic character, provided by the hydrophilic main chain, chitosan, and the hydrophobic cholanic acid bonded thereto, it serves as an anticancer drug carrier that enables the sustained release of drugs in a target-specific manner and thus has high anticancer activity. Therefore, the pharmaceutical formulation of the present invention is useful for treating diseases including cancer.

In addition, the present invention provides a method of preparing the cholanic acid-chitosan complex represented by Scheme 1, below, comprising the steps of:
(a) dissolving hydrophilic chitosan in a water-soluble solvent to provide a chitosan solution;
(b) dissolving hydrophobic cholanic acid in an organic solvent to provide a cholanic acid solution;
(c) adding in droplets the cholanic acid solution to the chitosan solution, and agitating the resulting reaction solution; and
(d) dialyzing the agitated reaction solution to remove non-reacted cholanic acid. The present invention provides a method of preparing the pharmaceutical formulation characterized in that hydrophobic anticancer drug paclitaxel is incorporated into the cholanic acid-chitosan complex, further comprising a step of physically incorporating the anticancer drug within the cholanic acid-chitosan complex.

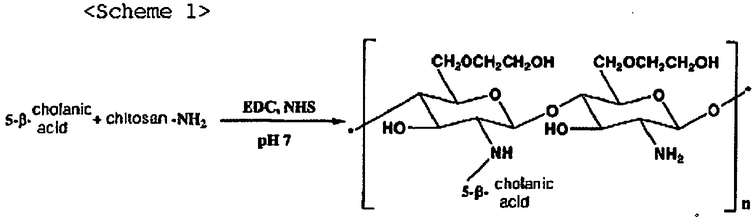

At step (a), hydrophilic chitosan is dissolved in a water-soluble solvent to provide a chitosan solution.

The hydrophilic glycol-chitosan, which serves as an anticancer drug carrier, may be any glycol-chitosan having a molecular weight ranging from 10³ to 10⁶ Da, which is a natural polymer, having excellent biodegradability and biocompatibility, and more preferably glycol chitosan having enhanced water solubility due to glycol groups introduced thereinto. The water-soluble solvent may be any water-soluble solvent capable of dissolving hydrophilic chitosan. Water is preferred.

The hydrophilic chitosan is preferably added in an amount from 830 to 840 mg per 100 ml of the water-soluble solvent.

At step (b), hydrophobic cholanic acid is dissolved in an organic solvent to provide a cholanic acid solution.

The hydrophobic cholanic acid is 5-β-cholanic acid, represented by Formula 1. The organic solvent is methanol.

The hydrophobic cholanic acid is added in an amount from 20 to 260 mg per 100 ml of the organic solvent.

At step (c), the cholanic acid solution prepared in step (b) is added in droplets to the chitosan solution prepared in step (a), and the resulting reaction solution is agitated.

If desired, a catalyst may be used for combining cholanic acid and chitosan. The catalyst is one or more selected from among 1-ethyl-3-(3-dimethyl-aminopropyl) carbodiimide (EDC) and N-hydrosuccinimide (NHS). When EDC is used as the catalyst, it is preferably used in a weight ratio of 0.9 to 1.1 to the cholanic acid used at step (b). NHS is preferably used in a weight ratio of 1.4 to 1.6 to EDC used.

After the catalyst is added to the reaction solution, the reaction solution may be agitated for 5 to 50 hours. If the reaction is carried out for shorter or longer periods of time, the bonding of cholanic acid and chitosan is not effectively achieved.

The preparation method of the present invention may further include, after step (c), a step of adding in droplets a solution of an anticancer drug to the reaction solution prepared at step (c) in order to physically incorporate the anticancer drug within the cholanic acid-chitosan complex forming self-aggregates according to the present invention.

The anticancer drug entrapped within the cholanic acid-chitosan complex is paclitaxel. Suitable solvents for dissolving anticancer drugs include all solvents capable of dissolving the anticancer drugs. Ethyl alcohol is preferred.

The present method of physically incorporating an anticancer drug within the cholanic acid-chitosan complex provides a maximum drug loading efficiency of 40% or greater, which is greatly enhanced compared to does chemical bonding, which provides a maximum loading efficiency of roughly 10%, thereby overcoming the limit of chemical bonding, resulting in greatly increased drug loading content.

At step (d), the agitated reaction solution is dialyzed to remove non-reacted cholanic acid, thereby yielding the cholanic acid-chitosan complex of the present invention.

Dialysis may be carried out by an conventional method. After non-reacted cholanic acid is removed, the reaction solution may further undergo freeze-drying to generate the cholanic acid-chitosan complex of the present invention.

### Mode for Invention

A better understanding of the present invention may be obtained through the following examples which are set forth to illustrate, but are not to be construed as the limit of the present invention.

### EXAMPLE 1: Preparation 1 of cholanic acid-chitosan complex

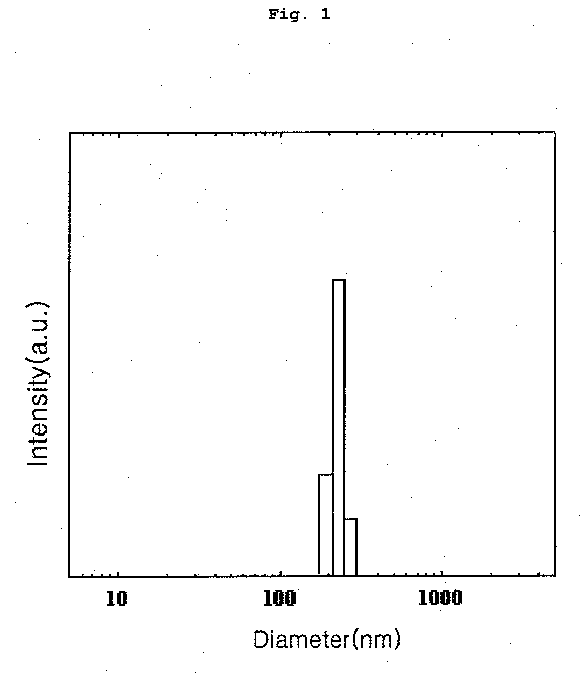

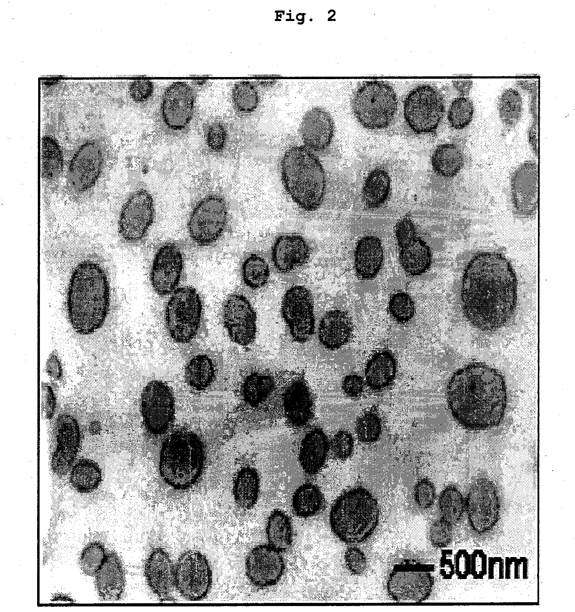

500 mg of glycol chitosan were dissolved in 60 ml of water, and mixed with 90 ml of methanol to provide a glycol chitosan solution. 260 mg of 5-β-cholanic acid were dissolved in 100 ml of methanol, and slowly added in droplets to the glycol chitosan solution. Then, 280 mg of 1-ethyl-3-(3-dimethyl-aminopropyl) carbodiimide (EDC) and 420 mg of N-hydrosuccinimide (NHS) were dissolved in 50 ml of methanol, added to the reaction solution, and agitated at room temperature for 24 hrs. The resulting reaction solution was dialyzed for 2 days to remove non-reacted 5-β-cholanic acid, and freeze-dried, thereby yielding a cholanic acid- chitosan complex according to the present invention. Light scattering analysis (FIG. 1) and transmission electron microscopic image (FIG. 2) resulted in the finding that a cholanic acid-chitosan complex was successfully prepared.

As shown in FIGS. 1 and 2, the cholanic acid-chitosan complex prepared was 200 to 800 nm in diameter.

### EXAMPLE 2: Preparation 2 of cholanic acid-chitosan complex

A cholanic acid-chitosan complex according to the present invention was prepared according to the same procedure as in Example 1, except that 5-β-cholanic acid and catalysts were used in different amounts. That is, 108.5 mg of 5-β-cholanic acid were dissolved in 100 ml of methanol, and slowly added in droplets to a glycol chitosan solution having the same concentration as in Example 1. 100 mg of EDC and 175 mg of NHS were dissolved in 50 ml of methanol and added to the reaction solution.

### EXAMPLE 3: Preparation 3 of 5-β-cholanic acid-chitosan complex

A cholanic acid-chitosan complex according to the present invention was prepared according to the same procedure as in Example 1, except that 5-β-cholanic acid and catalysts were used in different amounts. That is, 21.7 mg of 5-β-cholanic acid were dissolved in 100 ml of methanol, and slowly added in droplets to a glycol chitosan solution having the same concentration as in Example 1. 20 mg of EDC and 35 mg of NHS were dissolved in 50 ml of methanol and added to the reaction solution.

The changes in cholanic acid content of the cholanic acid-chitosan complexes prepared with the varying amount of cholanic acid in Examples 1 to 3 were examined in Test Example 1, below.

### EXAMPLE 4: Preparation 4 of 5-β-cholanic acid-chitosan complex

A cholanic acid-chitosan complex according to the present invention was prepared according to the same procedure as in Example 1, except that 5-β-cholanic acid and chitosan were allowed to react for 6 hrs.

### EXAMPLE 5: Preparation 5 of 5-β-cholanic acid-chitosan complex

A cholanic acid-chitosan complex according to the present invention was prepared according to the same procedure as in Example 1, except that 5-β-cholanic acid and chitosan were allowed to react for 12 hrs.

### EXAMPLE 6: Preparation 6 of 5-β-cholanic acid-chitosan complex

A cholanic acid-chitosan complex according to the present invention was prepared according to the same procedure as in Example 1, except that 5-β-cholanic acid and chitosan were allowed to react for 18 hrs.

### EXAMPLE7: Preparation 7 of 5-β-cholanic acid-chitosan complex

A cholanic acid-chitosan complex according to the present invention was prepared according to the same procedure as in Example 1, except that 5-β-cholanic acid and chitosan were allowed to react for 48 hrs.

The changes in cholanic acid content of the cholanic acid-chitosan complexes prepared for the varying reaction times of cholanic acid and chitosan in Examples 4 to 7 were examined in Test Example 1, below.

### EXAMPLES 8 to 12: Preparation of paclitaxel-physically incorporated 5-β-cholanic acid-chitosan complexes

Cholanic acid-chitosan complexes entrapping paclitaxel according to the present invention were prepared as follows. Paclitaxel was dissolved in ethyl alcohol in concentrations of 1%, 3%, 5%, 10% and 20%. 5 mg of a cholanic acid-chitosan complex according to the present invention was dissolved in a mixture of water and ethyl alcohol at a volume ratio of 1:1, and the resulting solution was slowly added in droplets to the paclitaxel solution, followed by gentle agitation for 24 hrs. Then, the resulting reaction solution was dialyzed using dialysis tubing having a molecular weight cut-off (MWCO) of 3,500 for 2 days in order to eliminate ethyl alcohol and paclitaxel not incorporated into the cholanic acid-chitosan complex. The dialyzed solution was freeze-dried, thereby yielding the cholanic acid-chitosan complexes entrapping paclitaxel according to the present invention.

### EXPERIMENTAL EXAMPLE 1: Evaluation of cholanic acid content of the cholanic acid-chitosan complexes of the present invention according to the preparation conditions

The changes in cholanic acid content of the cholanic acid-chitosan complexes prepared by varying the amount of cholanic acid or the reaction time of cholanic acid and chitosan in Examples 1 to 7 were examined, as follows.

5 mg of a 5-β-cholanic acid-chitosan complex was dissolved in 10 ml of 2% acetic acid, and 20 (µl of 0.1% toluidine blue was added thereto, and agitated. During agitation, a solution of N/400 potassium polyvinyl sulfate (PVSK) was dropped, and the addition thereof was terminated when the color of the solution changed. The cholanic acid content was determined by colloidal titration calculating the amount of PVSK added.

The results were given in Table 1, below.

**TABLE 1**

| Example | Amount of cholanic acid used | Reaction time (hrs) | Cholanic acid content of cholanic acid-chitosan complex |
|---|---|---|---|
| 1 | 260 | 24 | 12 |
| 2 | 108.5 | 24 | 5 |
| 3 | 21.7 | 24 | 1 |
| 4 | 260 | 6 | 12 |
| 5 | 260 | 12 | 12 |
| 6 | 260 | 18 | 12 |
| 7 | 260 | 48 | 12 |

As shown in Table 1, when a cholanic acid-chitosan complex was prepared by varying the amount of cholanic acid in Examples 1 to 3, its cholanic acid content increased when the amount of cholanic acid used was increased. Also, when a cholanic acid-chitosan complex was prepared by varying the reaction time of cholanic acid and chitosan in Examples 4 to 7, no change in the cholanic acid content was observed, indicating that the extended reaction time did not affect the cholanic acid content.

### EXPERIMENTAL EXAMPLE 2: Evaluation of the drug incorporation rates of the cholanic acid-chitosan complexes of the present invention

The cholanic acid-chitosan complexes into which a drug was physically incorporated according to the present invention were evaluated for their drug loading efficiencies, as follows.

Drug loading efficiencies were measured for the 5-β-cholanic acid-chitosan complexes prepared in Examples 8 to 12 by checking the amount of paclitaxel that was used and the amount of paclitaxel that was actually-loaded. The diameter of the 5-β-cholanic acid-chitosan complex entrapping paclitaxel was measured using a commonly used light scattering method.

The results were given in Table 2, below.

**TABLE 2**

| Example | Amount of paclitaxel used (wt%) | Amount of loaded paclitaxel (wt%) | Loading efficiency(%) | d (nm) (ja2/T\|2) |
|---|---|---|---|---|
| 1 | - | - | - | 200 (0.12) |
| 8 | 1 | 0.94±0.05 | 94±5 | 220 (0.13) |
| 9 | 3 | 2.7610.09 | 92±3 | 240 (0.19) |
| 10 | 5 | 4.55±0.25 | 91±6 | 310 (0.13) |
| 11 | 10 | 9+0.50 | 90+5 | 400 (0.13) |

As shown in Table 2, when a 5-β-cholanic acid- chitosan complex was prepared according to the preparation method of the present invention, the incorporation of paclitaxel into the complex increased when the amount of paclitaxel used was increased. The present physical method provided a drug loading efficiency of 90% or greater, which was much higher than the drug loading efficiency realized by chemical bonding.

In addition, the diameter of the 5-β-cholanic acid-chitosan complex increased with the increasing incorporation of paclitaxel.

### EXPERIMENTAL EXAMPLE 3: Evaluation of the release patterns of paclitaxel entrapped in the 5-β-cholanic acid-chitosan complex

The paclitaxel-entrapped cholanic acid-chitosan complex prepared in Example 11 was dispersed in water in a concentration of 2 mg/ml. 500 ial of the dispersion was placed inside cellulose dialysis tubing (molecular weight cut-off: 12,000-14,000), sealed, and immersed in water at 37°C with agitation at 150 rpm. The water was collected at given time points and assessed using HPLC for the amount of paclitaxel that was released.

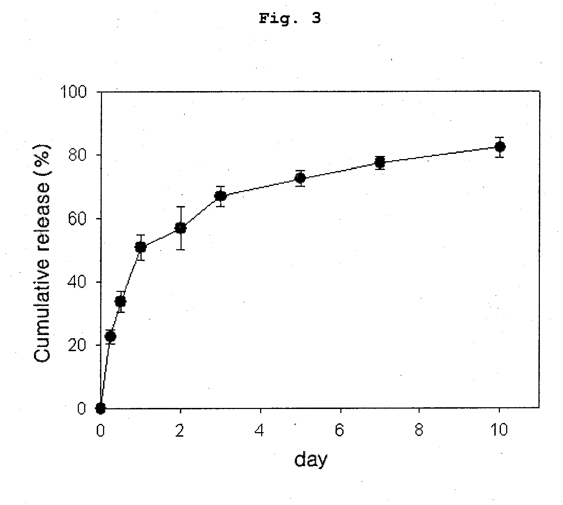

The results were given in FIG. 3.

As shown in FIG. 3, the cumulative release of paclitaxel increased over time, indicating that the release of paclitaxel was sustained.

### EXPERIMENTAL EXAMPLE 4: Evaluation of the anticancer effect of the paclitaxel-entrapped cholanic acid-chitosan complex

The paclitaxel-entrapped cholanic acid-chitosan complex prepared in Example 11 was assessed for its anticancer effect using C57BL/6 mice suffering from melanoma, as follows.

The cholanic acid-chitosan complex according to the present invention or the paclitaxel-containing cholanic acid-chitosan complex was administered to mice implanted with B16F10 murine melanoma cells in a dose of 20 mg/kg or 50 mg/kg for a period of 21 days. Then, the experimental animals were weighed, and the volume of tumors excised from the animals was measured. Cremophor EL (polyethoxylated castor oil derivatives, BASF) containing paclitaxel was used as a comparative group, and physiological saline was used as a negative control.

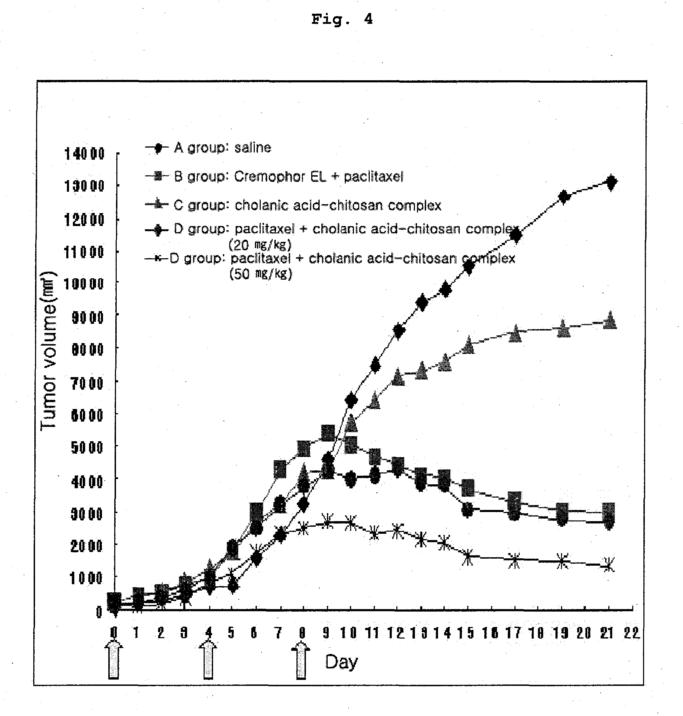

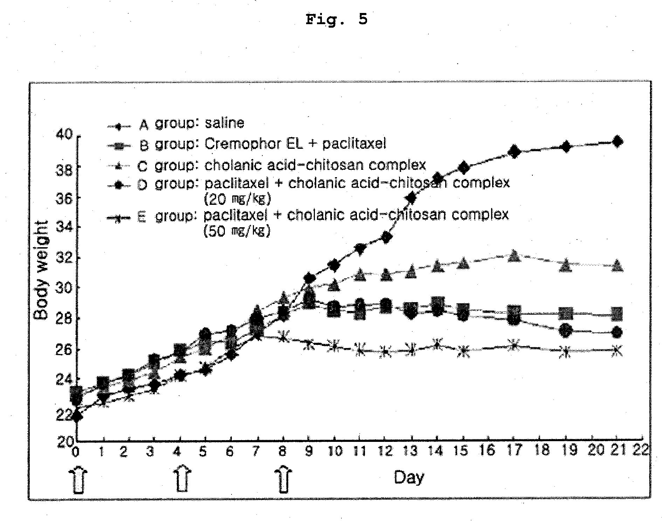

The results were given in FIGS. 4 and 5.

As shown in FIG. 4, the tumors negative control mice that received only physiological saline were found to increase in volume over time. In mice that received the cholanic acid-chitosan complex or paclitaxel-entrapped cholanic acid-chitosan complex according to the present invention, or Cremophor EL, containing paclitaxel, the tumor size seldom changed after Day 9. Also, a smaller increase in the tumor size was observed when the paclitaxel- entrapped cholanic acid-chitosan complex was administered at a higher dose.

As shown in FIG. 5, results similar to the results shown in FIG. 4 were obtained when the animals were weighed. Taken together, these results indicate that the paclitaxel-entrapped cholanic acid-chitosan complex according to the present invention enables the sustained release of paclitaxel and thus may exert anticancer effects for prolonged periods of time.

### Industrial Applicability

As described hereinbefore, the pharmaceutical formulation characterized in that hydrophobic anticancer drug paclitaxel is incorporated into a cholanic acid-chitosan complex, wherein the cholanic acid-chitosan complex forms self-aggregates, prolongs the drug release period, enhances the selectivity of the complex for the tumor tissue, and greatly increases drug loading content when a drug is incorporated into the self-aggregates, compared to chemical bonding, which limits drug incorporation. Thus, the pharmaceutical formulation of the present invention is useful for anticancer chemotherapy.

## Claims

1. A pharmaceutical formulation **characterized in that** paclitaxel is incorporated into 5-β-cholanic acid - glycol chitosan complex, wherein the 5-β-cholanic acid - glycol chitosan complex forms self-aggregates in an aqueous solution.

2. The pharmaceutical formulation according to claim 1, wherein the glycol chitosan has a mean molecular weight from 10³ to 10⁶ Da.

3. The pharmaceutical formulation according to claim 1, wherein the 5-β-cholanic acid-glycol chitosan complex with paclitaxel is 10nm to 800 nm in diameter.

4. A method of preparing the pharmaceutical formulation **characterized in that** paclitaxel is incorporated into the 5-β-cholanic acid -glycol chitosan complex that forms self-aggregates in an aqueous solution, comprising the steps of:
(a) dissolving glycol chitosan in a water-soluble solvent to provide a glycol chitosan solution;
(b) dissolving 5-β-cholanic acid in methanol to provide a 5-β-cholanic acid solution, wherein the glycol cholanic acid is added in an amount from 20 to 260 mg per 100 ml of methanol;
(c) adding in droplets the 5-β-cholanic acid solution to the glycol chitosan solution, and agitating a resulting reaction solution;
(d) dialyzing the agitated reaction solution to remove non-reacted 5-β-cholanic acid and accordingly prepare a 5-β-cholanic acid -glycol chitosan complex.

5. The method of preparing the pharmaceutical formulation according the claim 4, wherein the glycol chitosan at step (a) has a mean molecular weight from 10³ to 10⁶ Da.

6. The method of preparing the pharmaceutical formulation according to claim 4, wherein the water-soluble solvent at step (a) is water.

7. The method of preparing the pharmaceutical formulation according the claim 4, wherein the glycol chitosan at step (a) is added in an amount from 830 to 840 mg per 100 ml of the water-soluble solvent.

## Patentansprüche

1. Pharmazeutische Formulierung, **dadurch gekennzeichnet, dass** Paclitaxel in einen 5-β-Cholansäure-Glycolchitosan-Komplex integriert ist, wobei der 5-β-Cholansäure-Glycolchitosan-Komplex in wässriger Lösung Selbstaggregate bildet.

2. Pharmazeutische Formulierung nach Anspruch 1, wobei das Glycolchitosan ein mittleres Molekulargewicht von 10³ bis 10⁶ Da aufweist.

3. Pharmazeutische Formulierung nach Anspruch 1, wobei der 5-β-Cholansäure-Glycolchitosan-Komplex mit Paclitaxel einen Durchmesser von 10 nm bis 800 nm aufweist.

4. Verfahren zur Herstellung der pharmazeutischen Formulierung, **dadurch gekennzeichnet, dass** Paclitaxel in den 5-β-Cholansäure-Glycolchitosan-Komplex integriert wird, welcher in wässriger Lösung Selbstaggregate bildet, wobei das Verfahren die folgenden Schritte umfasst:
(a) Lösen von Glycolchitosan in einem wasserlöslichen Lösungsmittel zum Erzeugen einer Glycolchitosanlösung;
(b) Lösen von 5-β-Cholansäure in Methanol zum Erzeugen einer 5-β-Cholansäurelösung, wobei die Glycolcholansäure in einer Menge von 20 bis 260 mg pro 100 ml Methanol hinzugegeben wird;
(c) tröpfchenweises Zugeben der 5-β-Cholansäurelösung zu der Glycolchitosanlösung und Rühren einer resultierenden Reaktionslösung;
(d) Dialysieren der gerührten Reaktionslösung zum Entfernen nichtreagierter 5-β-Cholansäure und folglich Herstellen eines 5-β-Cholansäure-Glycolchitosan-Komplexes.

5. Verfahren zur Herstellung der pharmazeutischen Formulierung nach Anspruch 4, wobei das Glycolchitosan in Schritt (a) ein mittleres Molekulargewicht von 10³ bis 10⁶ Da aufweist.

6. Verfahren zur Herstellung der pharmazeutischen Formulierung nach Anspruch 4, wobei das wasserlösliche Lösungsmittel in Schritt (a) Wasser ist.

7. Verfahren zur Herstellung der pharmazeutischen Formulierung nach Anspruch 4, wobei das Glycolchitosan in Schritt (a) in einer Menge von 830 bis 840 mg pro 100 ml des wasserlöslichen Lösungsmittels zugegeben wird.

## Revendications

1. Formulation pharmaceutique **caractérisée en ce que** du paclitaxel est incorporée dans un complexe acide 5-β-cholanique/glycol chitosan, dans laquelle le complexe acide 5-β-cholanique/glycol chitosan forme des auto-agrégats dans une solution aqueuse.

2. Formulation pharmaceutique selon la revendication 1, dans laquelle le glycol chitosan a un poids moléculaire moyen de 10³ à 10⁶ Da.

3. Formulation pharmaceutique selon la revendication 1, dans laquelle le complexe acide 5-β-cholanique/glycol chitosan avec du paclitaxel a un diamètre de 10 nm à 800 nm.

4. Procédé de préparation de la formulation pharmaceutique **caractérisée en ce que** du paclitaxel est incorporé dans le complexe acide 5-β-cholanique/glycol chitosan formant des auto-agrégats dans une solution aqueuse, comprenant les étapes de :
(a) dissoudre du glycol chitosan dans un solvant soluble dans l'eau afin de produire une solution de glycol chitosan ;
(b) dissoudre de l'acide 5-β-cholanique dans du méthanol afin de produire une solution d'acide 5-β-cholanique, dans laquelle l'acide cholanique glycolique est ajouté en une quantité de 20 à 260 mg pour 100 ml de méthanol ;
(c) ajouter en gouttelettes la solution d'acide 5-β-cholanique à la solution de glycol chitosan et agiter une solution de réaction résultante ;
(d) dialyser la solution de réaction agitée afin d'éliminer l'acide 5-β-cholanique n'ayant pas réagi et par conséquent préparer un complexe acide 5-β-cholanique/glycol chitosan.

5. Procédé de préparation de la formulation pharmaceutique selon la revendication 4, dans lequel le glycol chitosan à l'étape (a) a un poids moléculaire moyen de 10³ à 10⁶ Da.

6. Procédé de préparation de la formulation pharmaceutique selon la revendication 4, dans lequel le solvant soluble dans l'eau à l'étape (a) est de l'eau.

7. Procédé de préparation de la formulation pharmaceutique selon la revendication 4, dans lequel le glycol chitosan à l'étape (a) est ajouté en une quantité de 830 à 840 mg pour 100 ml de solvant soluble dans l'eau.
